# NEUE EUROPÄISCHE PATENTSCHRIFT

(11) **EP 0 321 918 B2**
(45) Veröffentlichungstag und Bekanntmachung des Hinweises auf die Entscheidung über den Einspruch: **14.04.1999**
(45) Hinweis auf die Patenterteilung: 23.03.1994
(21) Anmeldenummer: 88121274.0
(22) Anmeldetag: 20.12.1988
(51) Int. Cl.: C12P 7/62

(54) **Verfahren zur enzymatischen Racematspaltung von racemischen Alkoholen mit/in vinylestern durch Umesterung**
Process for the enzymatic racemate cleavage of racemic alcohols with/in vinyl esters by transesterification
Procédé enzymatique de dédoublement de racémates d'alcools racémiques avec ou dans des esters vinyliques par transestérification.

(30) Priorität: 23.12.1987 DE 3743824
(43) Veröffentlichungstag der Anmeldung: 28.06.1989
(73) Patentinhaber: Clariant GmbH, 65929 Frankfurt am Main (DE)
(72) Erfinder: Keller, Reinhold, Dr., D-6232 Bad Soden am Taunus (DE); Holla, Wolfgang, Dr., D-6238 Hofheim am Taunus (DE); Fülling, Gerd, Dr., D-6230 Frankfurt am Main 80 (DE); Prof. Dr. Manfred Schneider, 5600 Wuppertal (DE)

(56) Entgegenhaltungen:
- EP-A- 0 191 217
- EP-A- 0 266 217
- TETRAHEDRON LETTERS, Band 28, Nr. 9, 1987, Seiten 953-954, Pergamon Journals Ltd, GB; M. DEGUEIL-CASTAING et al.: "Enzymatic reactions in organic synthesis: 2-ester interchange of vinyl esters"
- J. AM. CHEM. SOC., Band 110, 1988, Seiten 7200-7205, American Chemical Society;Y.-F. WANG et al.: "Lipase-catalyzed irreversible transesterifications usingenol esters as acylating reagents: preparative enantio- and regioselectivesyntheses of alcohols, glycerol derivatives, sugars, and organometallics"
- J. BIOCHEM., Band 104, Nr. 5, 1988, Seiten 681-682; T. NISHIO et al.:"Enzymatic transesterification with the lipase from Pseudomonas fragi 22.39 Bin a non-aqueous reaction system"
- J.Org.Chem. 53, 1988, 3127-3129. Y.Wang and C.Wong.
- Tetrahedron Letters 28, 1987, 953-954. M Degueil-Castaing.
- Lipids 23, 1988, 955-960. P E Sonnet and G G Moore

## Beschreibung

Die Erfindung betrifft ein Verfahren zur enzymatischen Racematspaltung von racemischen Alkoholen mit bzw. in Vinylestern durch Umesterung, wobei der Vinylester in ein Keton bzw. einen Aldehyd und einen "Säurerest" gespalten wird und wobei der verbliebene Säurerest mit einem hinzugegebenen racemischen Alkohol enantioselektiv einen Ester bildet und allein ein Enantiomeres des Alkohols unverändert zurückbleibt. Ester und nicht umgesetzter Alkohol und somit beide enantiomeren Alkohole können leicht voneinander getrennt werden. Das zweite Enantiomere des Alkohols kann gegebenenfalls durch Spaltung des Esters gewonnen werden.

Viele optisch aktive Alkohole sind wichtige chirale Vorstufen von biologisch aktiven Substanzen (Arzneimittel, Naturstoffe, Pflanzenschutzmittel) und ein wirtschaftliches Herstellungsverfahren ist deshalb von großer Bedeutung. Einige pharmakologische Wirkstoffe, deren Darstellung mit dem erfindungsgemäßen Verfahren erleichtert wird, sind beispielsweise NSAIDs (non-steroidal-antiinflammatory-drugs) wie Ibuprofen und Naproxen, Betablocker wie Nifenalol und Penbutolol, Bronchospasmolytica wie Tolubuterol und Bitolterol, Antimycotica wie Tioconazol, Pyrethroide wie Allethrine, darüber hinaus Tetramisol, Tetrahydrozolin, (R)-(-)-Tomoxetine und (S)-(+)-Fluoxetine sowie Prostaglandine und Kohlenhydrate.

Es ist bekannt, daß man Vinylester unter Hinzugabe von Alkoholen in Gegenwart von Lösungsmitteln wie Tetrahydrofuran unter enzymatischer Katalyse umestern kann (M. Degueil-Castaing et al. Tetrahedron Letters. Vol. 28, No. 9, Seiten 953-954, 1987). Als Enzym wurde PPL (Pig Pancreatic Lipase) verwendet. Eine Stereoselektivität wurde bei der Reaktion nicht beobachtet.

Atushi Makita et al. beschreiben in Tetrahedron Letters, Vol. 28, No. 7, Seiten 805-808, 1987 den Einsatz von Lipase P zur "Lactonisierung" von ω-Hydroxycarbonsäuremethylestern in organischen Lösungsmitteln wie Cyclohexan, Chloroform, Hexan, Heptan, Benzol, Toluol oder Dimethylsulfoxid.

In der deutschen Patentanmeldung P 36 24 703.0 wurde vorgeschlagen, aus prochiralen Diolen durch Umsetzung mit Vinylacetat in Gegenwart von Hydrolasen chirale Verbindungen optisch rein darzustellen. Dies gelingt durch selektive Veresterung nur einer der beiden enantiotopen primären OH-Gruppen.

Es wurde nun überraschend gefunden, daß sich racemische Alkohole enzymatisch trennen lassen, indem man sie einer selektiven enzym katalysierten Umesterungsreaktion mit Vinylestern unterwirft, wobei auf Lösungsmittel verzichtet werden kann. Als Enzyme können Lipasen aus Schweineleber und Schweinepankreas sowie aus Mikroorganismen, wie Candida, Mucor, Rhizopus, Penicillium und insbesondere aus Pseudomonas spec., wie Lipase P und Lipase FP (Amano, Japan) eingesetzt werden.

Dies war um so überraschender, als bisher angenommen wurde, daß die freigesetzten Carbonylverbindungen (Aldehyde oder Ketone) mit den Lipasen reagieren und diese desaktivieren.

Die Erfindung betrifft somit:
Ein Verfahren zur enzymatischen Racematspaltung von racemischen Alkoholen, wobei man einen Vinylester der Formel I, 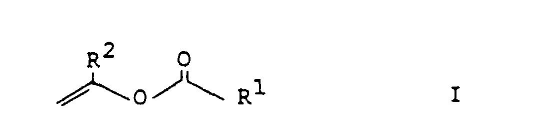 in der
- R¹: Wasserstoff, C₁-C₁₈-Alkyl, welches gegebenenfalls mit Halogen substituiert ist, Phenyl oder (C₁-C₃)-Alkoxy-(C₁-C₄)-Alkyl
und
- R²: Wasserstoff oder Methyl bedeutet,

in Gegenwart von Lipasen aus Schweineleber, Schweinepankreas sowie aus Mikroorganismen, wie Candida, Mucor, Rhizopus, Penicillium, Aspergillus und Pseudomonas umsetzt mit einem Alkohol der Formeln 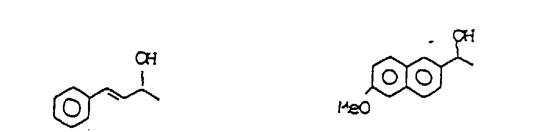 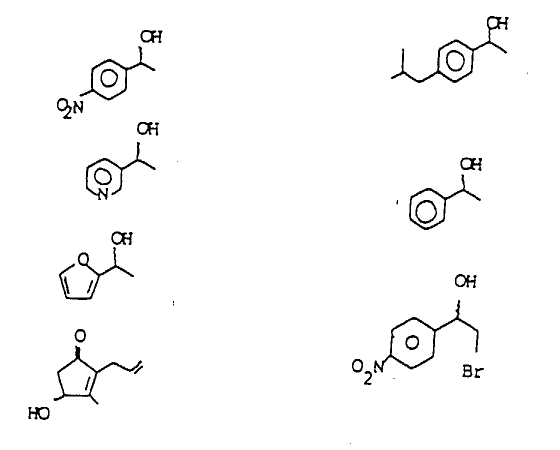 und anschließend den entstandenen optisch reinen Alkohol isoliert und gegebenenfalls das andere Enantiomere aus dem verbliebenen Ester gewinnt.

Unter Halogenen werden Fluor, Chlor, Brom und Jod, insbesondere Chlor und Brom verstanden. Der gegebenenfalls substituierte Rest R¹ ist bevorzugt monosubstituiert.

Alkyl- reste mit 3 und mehr Kohlenstoffatomen können sowohl geradkettig als auch verzweigt sein.

Das erfindungsgemäße Verfahren weist gegenüber herkömmlichen Verfahren zur Racematspaltung von Alkoholen folgende Vorteile auf:
a) Zumindest ein Enantiomeres ist immer (optisch) hochrein, meist sogar beide;
b) Das eingesetzte Enzym kann leicht zurückgewonnen werden;
c) Die Trennung des bei der Spaltung entstandenen "Esters" von dem "Alkohol" ist sehr einfach, im Idealfall lassen sich beide destillativ trennen;
d) Verzicht auf ein zusätzliches Lösungsmittel;
e) Hoher und schneller Umsatz bei einem Einsatz von vergleichsweise niedrigen Enzymmengen.

Bei dem erfindungsgemäßen Verfahren wird der Vinylester der Formel I in Carbonsäure und den entsprechenden Vinylalkohol gespalten. Der Vinylalkohol lagert sich sofort um in ein Keton bzw. einen Aldehyd. Eine Rückreaktion ist somit vollständig unterbunden. Unter der katalytischen Wirkung der genannten Lipasen wird auf diese Weise schnell und in hohen Ausbeuten selektiv ein Enantiomeres aus dem racemischen Alkohol verestert, das andere Enantiomere bleibt unverändert im Reaktionsgemisch.

Besonders gut eignet sich das erfindungsgemäße Verfahren zur Spaltung von solchen Alkoholen, die in β-Position zur OH-Gruppe eine C-C-Doppelbindung aufweisen.

Bei dem erfindungsgemäßen Verfahren geht man am besten so vor, daß man den Vinylester der Formel I, bevorzugt Vinylacetat oder Chloressigsäurevinylester vorlegt und zu dieser Lösung sowohl das Enzym als auch den zu spaltenden Alkohol hinzugibt. Als Enzym wird bevorzugt eine Lipoprotein-Lipase aus Pseudomonas spec., insbesondere Lipase P oder Lipase FP, die käuflich erhältlich sind, (Amano Pharmaceuticals, Nagoya, Japan) eingesetzt. Darüber hinaus kann das Enzym auch in immobilisierter Form eingesetzt werden, wobei hierzu alle gängigen Immobilisierungsmethoden und Träger in Frage kommen. Immobilisiertes Enzym und freies Enzym können auch im Säulenverfahren eingesetzt werden. Die Enzymmenge wird in Abhängigkeit von der Größe des Ansatzes, von der Reaktivität des Alkohols, von der anzustrebenden Reaktionszeit und von der Art des Enzyms (frei oder fixiert) frei gewählt und ist im Einzelfall durch einfache Vorversuche leicht zu bestimmen. Der zu spaltende Alkohol wird in Konzentrationen von 1 % bis 200 %, bevorzugt von 10 % bis 40 % - bezogen auf das Volumen des eingesetzten Vinyl-bzw. Methylvinylester - eingesetzt. In manchen Fällen ist es sogar möglich, Vinylester nur in stöchiometrischen Mengen - bezogen auf ein Enantiomeres - vorzulegen.

Die Reaktionstemperatur beträgt -10°C bis 100°C, bevorzugt 15°C bis 50°C. Es ist zweckmäßig, die Lösung während der Reaktion zu rühren. Die Reaktionszeiten schwanken je nach eingesetztem racemischem Alkohol und in Abhängigkeit von der Enzymmenge zwischen wenigen Stunden und bis zu 4 Wochen; liegen jedoch in der überwiegenden Zahl der Fälle zwischen 3 Stunden und 3 Tagen.

Die Vinyl- bzw. Methylvinylester der Formel I sind, sofern nicht käuflich, auf einfache Weise herstellbar, beispielsweise durch Umesterung von Vinylacetat mit den entsprechenden Carbonsäuren.

Die racemischen Alkohole erhält man, sofern sie nicht käuflich sind, z.B. durch Reduktion aus den entsprechenden Ketonen, die meist käuflich sind, oder durch α-Bromierung entsprechender Ketone mit anschließender Reduktion zum Alkohol. Andere nicht käufliche Alkohole oder Ketone lassen sich nach literaturbekannten Verfahren einfach herstellen, beispielsweise über Grignard- oder andere gängige Additionsreaktionen.

Die bei dem erfindungsgemäßen Verfahren entstehenden Produkte Acetaldehyd bzw. Aceton, Carbonsäureester und Alkohol lassen sich auf bekannte Art und Weise trennen. Im Idealfall erhält man den nicht umgesetzten Alkohol durch destillative Trennung. Ist eine destillative Trennung nicht möglich, so erfolgt die Trennung chromatographisch oder per Extraktion oder Kristallisation. Eine Möglichkeit, die Siedepunktdifferenz zwischen dem enzymatisch gebildeten niederen Ester und dem nicht umgesetzten Alkohol zu erhöhen, besteht darin, anstelle von Vinylester den entsprechenden α-Halogen-carbonsäurevinylester einzusetzen (z. B. α-Chloressigsäurevinylester).

Soll auch das andere Enantiomere rein dargestellt werden, so isoliert man zunächst den Ester und spaltet diesen auf bekannte Weise in Säure und Alkohol.

So erhält man Alkohole mit Enantiomerenreinheiten von über 95 % (ee-Wert).

Das für die Reaktionen verwendete Enzym läßt sich leicht durch Filtration zurückgewinnen.

In den nachfolgenden Beispielen wird die Erfindung detailliert erläutert.

### Beispiel

### Allgemeine Arbeitsvorschrift

Der racemische Alkohol wird in Vinylester gelöst bzw. suspendiert. Dazu gibt man Lipase P (bzw. Lipase FP) in freier oder fixierter Form und rührt bei der in Tabelle 1 angegebenen Temperatur. Nach Beendigung der Reaktion filtriert man das Enzym (sowohl das freie als auch das fixierte Enzym ist wiederverwendbar) ab, engt die verbleibende Lösung im Vakuum zur Trockne ein und trennt das als Rückstand verbleibende Alkohol/Ester-Gemisch durch Säulenchromatographie an Kieselgel, bzw. durch Extraktion, Kristallisation oder Destillation.

In Tabelle 1 sind die erhaltenen Produkte, die variablen Verfahrensparameter (Enzymmenge, Alkoholmenge, Vinylestermengen, Reaktionstemperatur, Reaktionszeit) sowie Produktcharakteristika und chemische Ausbeute angegeben.

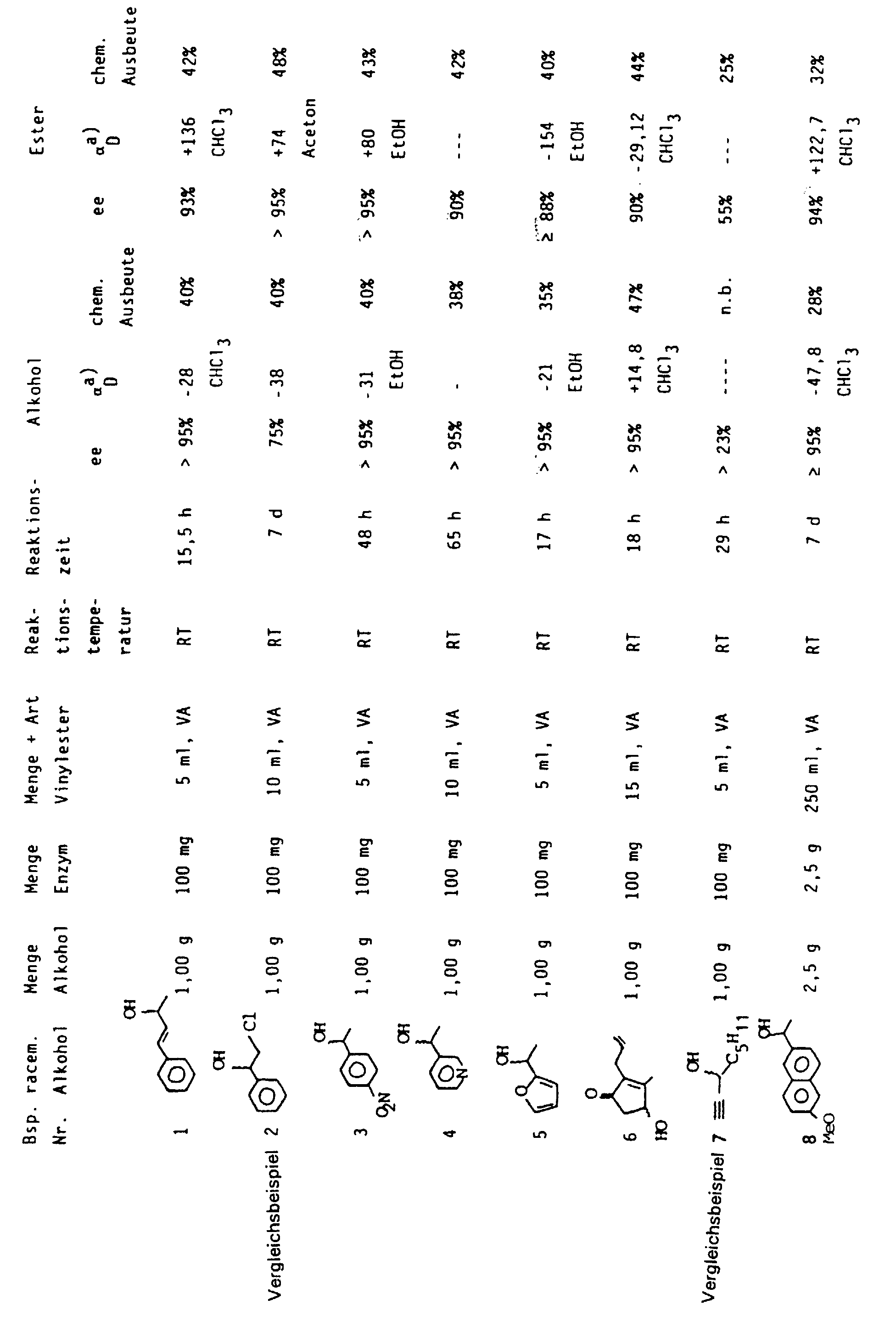

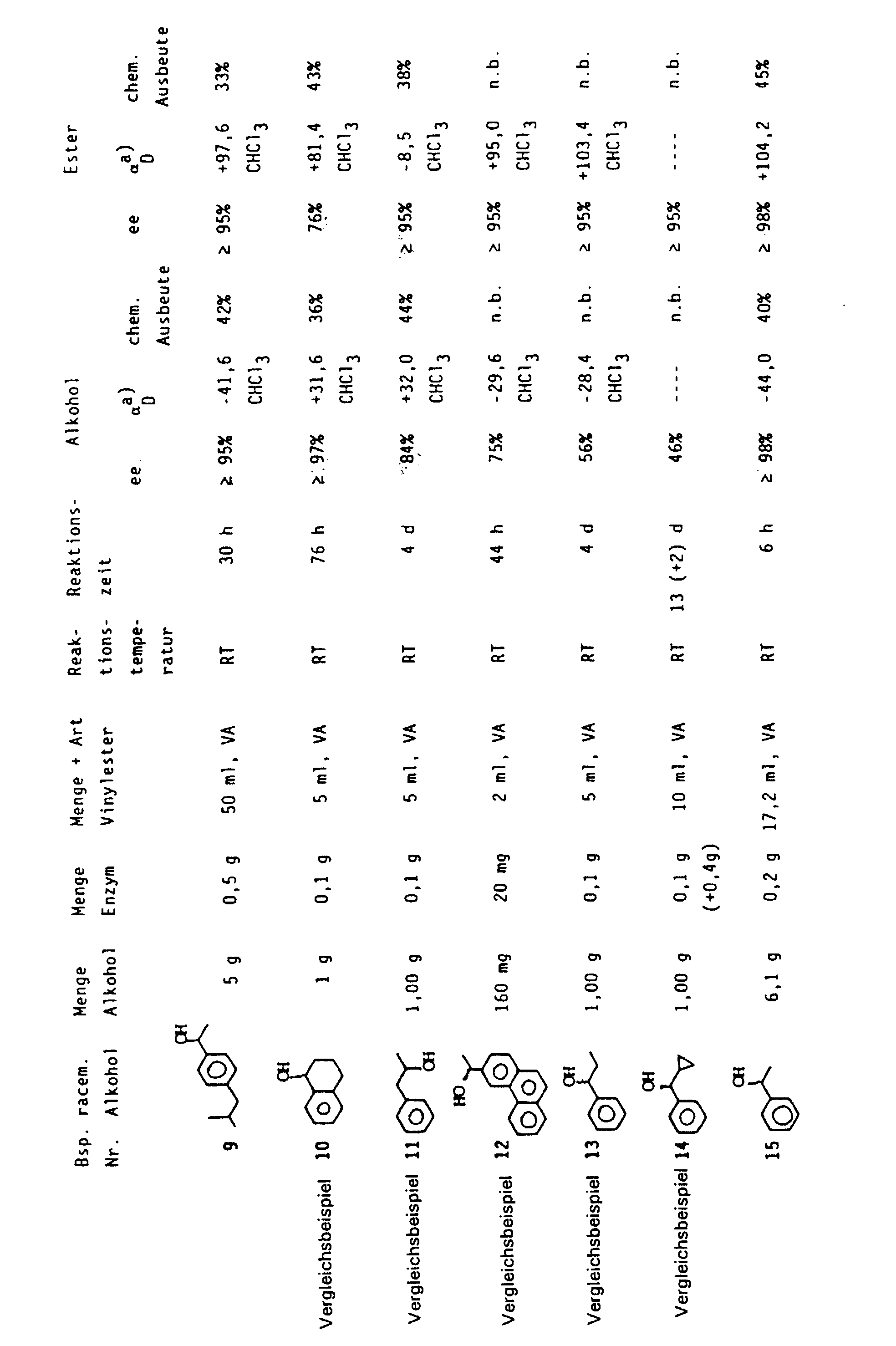

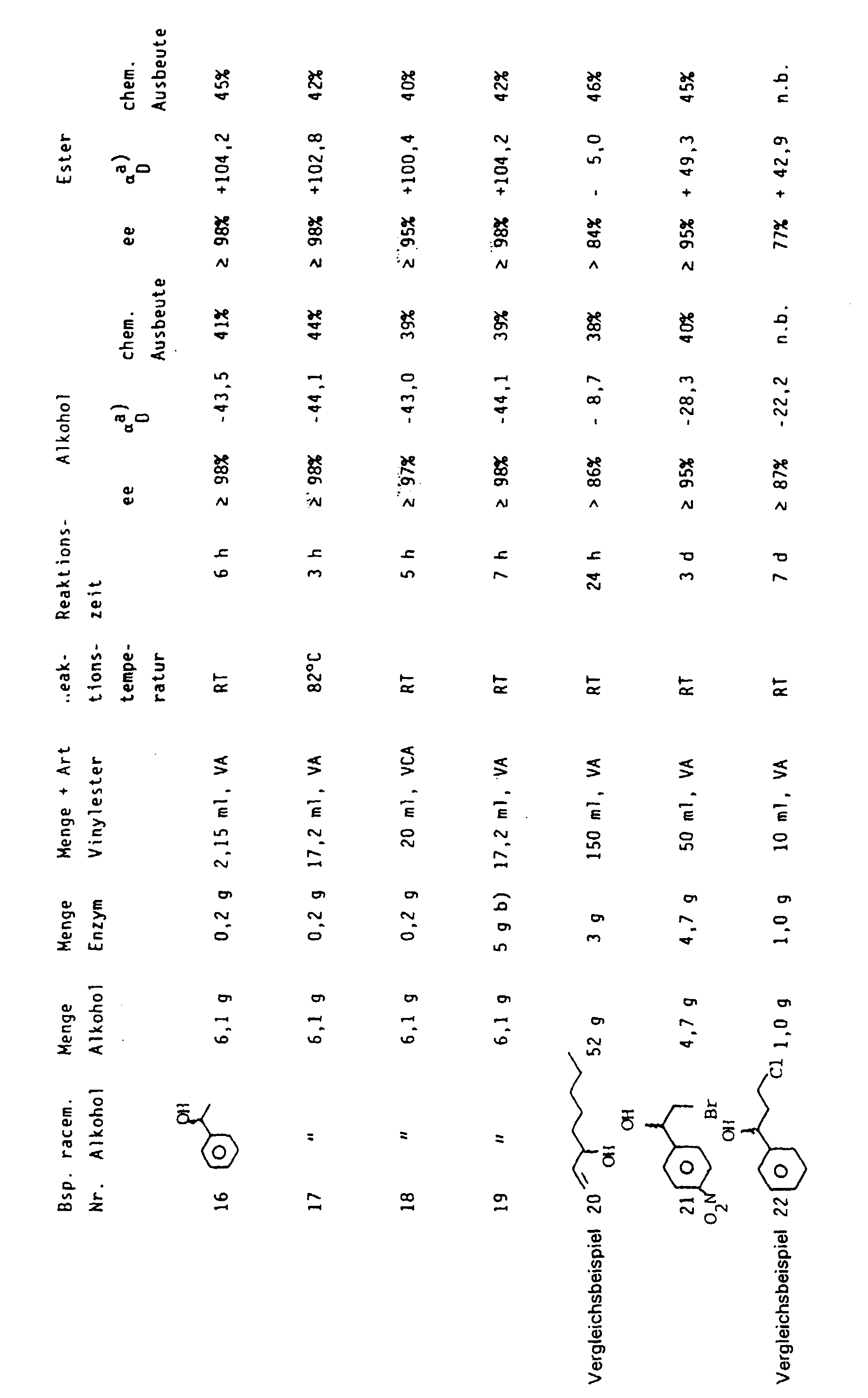

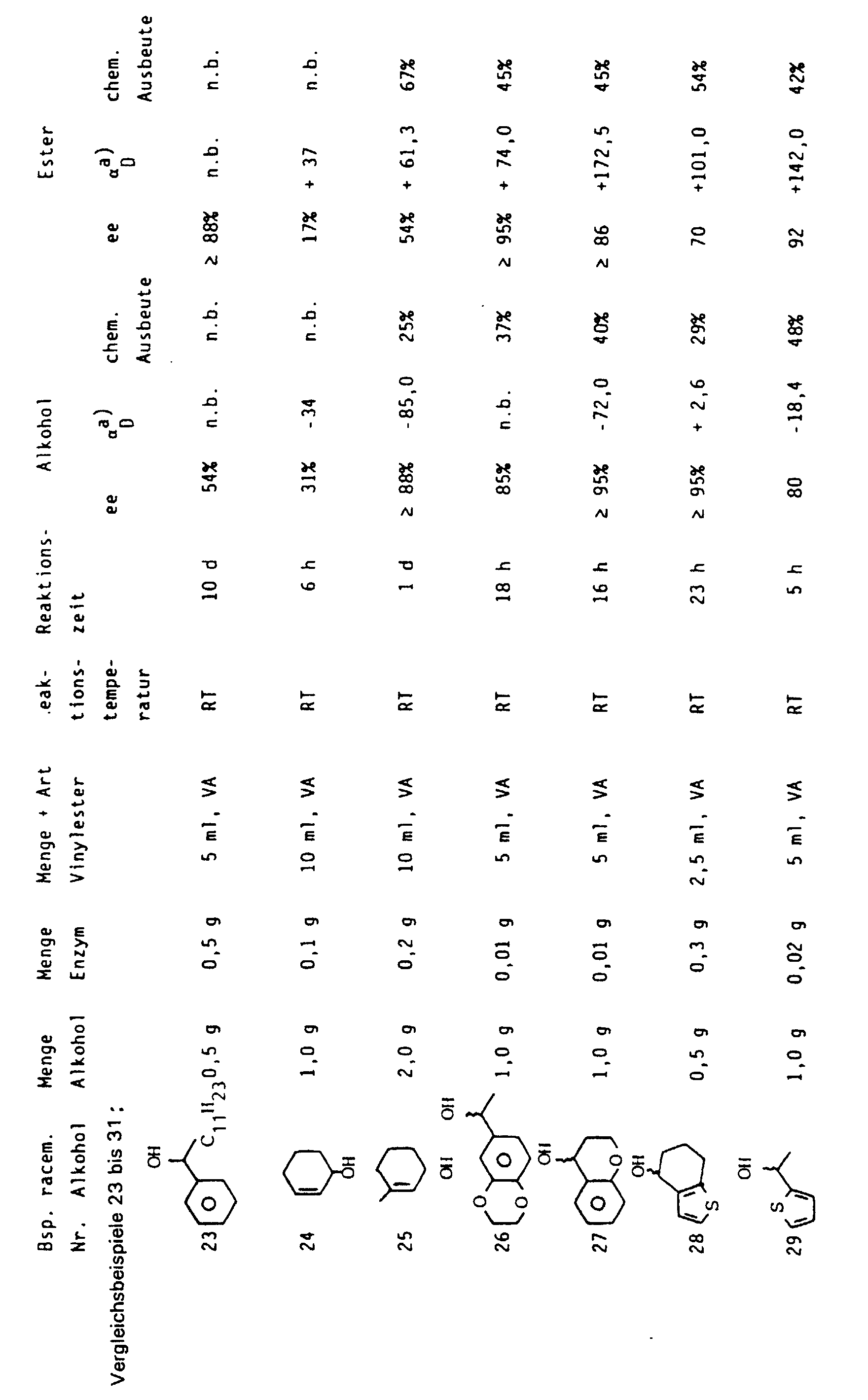

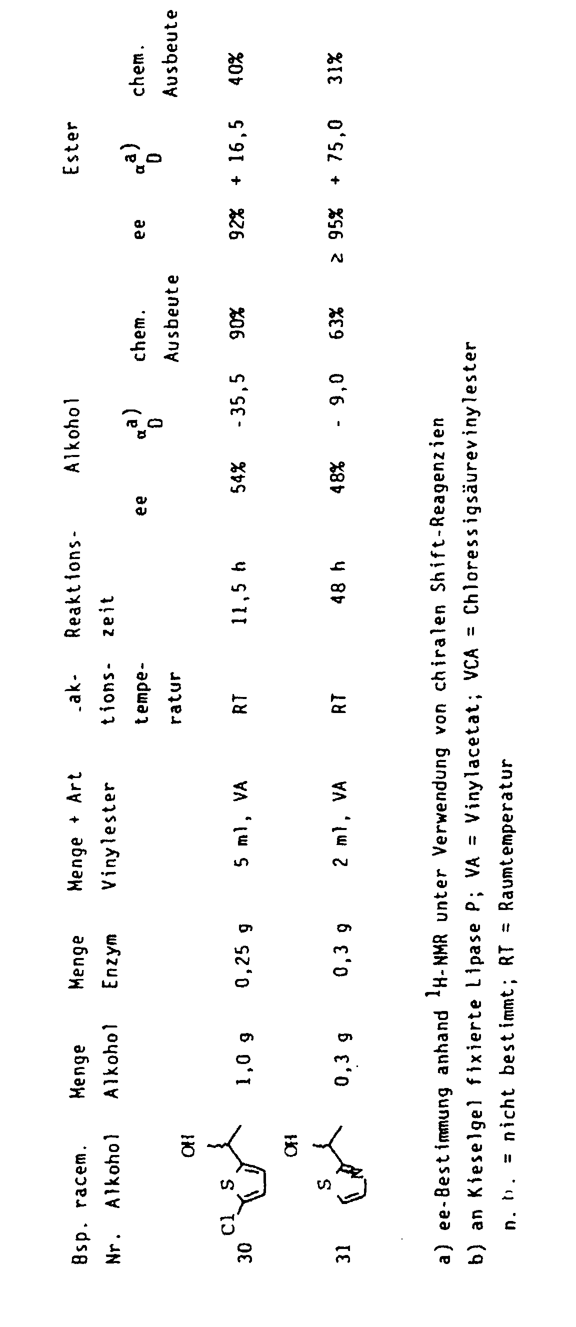

## Patentansprüche

1. Verfahren zur enzymatischen Racematspaltung von racemischen Alkoholen, dadurch gekennzeichnet, daß man einen Vinylester der Formel I, 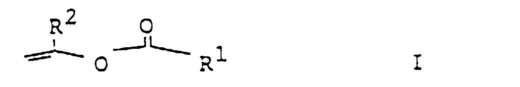 in der
R¹ Wasserstoff, C₁-C₁₈-Alkyl, welches gegebenenfalls mit Halogen substituiert ist, Phenyl oder C₁-C₃-Alkoxy-C₁-C₄-Alkyl
und
R² Wasserstoff oder Methyl bedeutet,
in Gegenwart von Lipasen aus Schweineleber, Schweinepankreas sowie aus Mikroorganismen, wie Candida, Mucor, Rhizopus, Penicillium, Aspergillus und Pseudomonas umsetzt mit einem Alkohol der Formeln 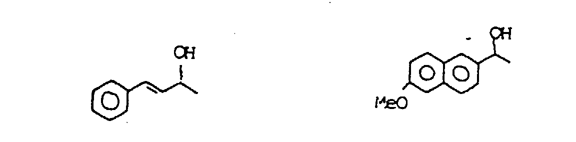 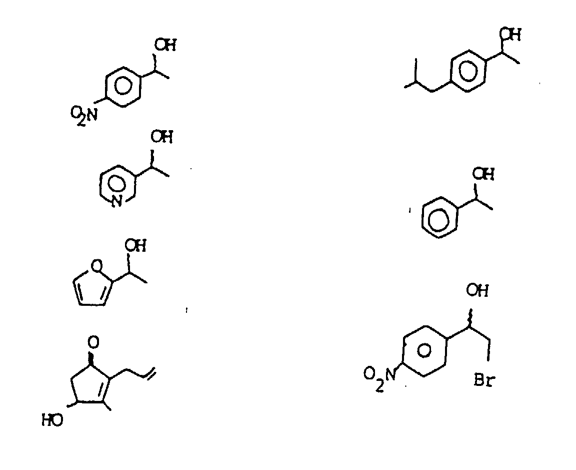 und anschließend den entstandenen optisch reinen Alkohol isoliert und gegebenenfalls das andere Enantiomere aus dem verbliebenen Ester gewinnt.

2. Verfahren nach Anspruch 1, dadurch gekennzeichnet, daß
R¹ bedeutet C₁-C₄-Alkyl, welches gegebenenfalls chlorsubstituiert ist.

3. Verfahren nach Anspruch 1 oder 2, dadurch gekennzeichnet, daß
R¹ bedeutet Methyl oder Chlormethyl

4. Verfahren nach einem oder mehreren der Ansprüche 1 bis 3, dadurch gekennzeichnet, daß Lipase von Pseudomonas eingesetzt wird.

## Claims

1. A method for the enzymatic racemate resolution of racemic alcohols, which comprises a vinyl ester of the formula I 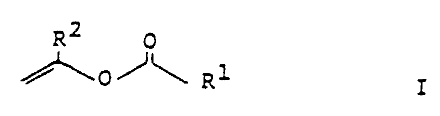 in which
R¹ denotes hydrogen, C₁-C₁₈-alkyl which is optionally halogen-substituted, phenyl or C₁-C₃-alkoxy-C₁-C₄-alkyl, and
R² denotes hydrogen or methyl,
being reacted, in the presence of lipases from pig liver, pig pancreas and from microorganisms such as Candida, Mucor, Rhizopus, Penicillium, Aspergillus and Pseudomonas, with an alcohol of the formulae 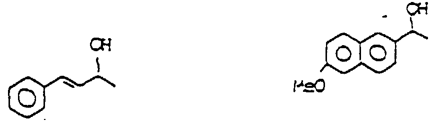 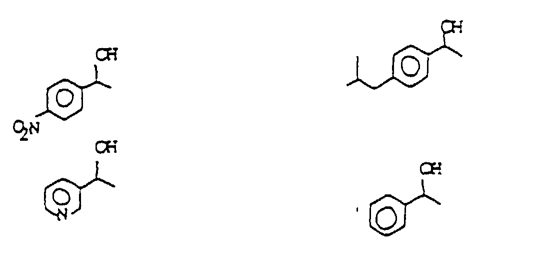 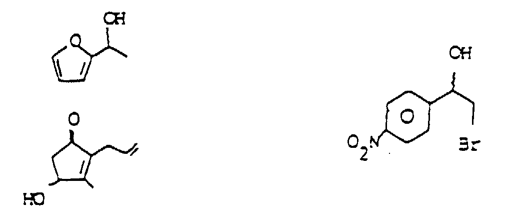 and subsequently isolating the resulting optically pure alcohol and, where appropriate, obtaining the other enantiomer from the remaining ester.

2. The method as claimed in claim 1, wherein
R¹ denotes C₁-C₄-alkyl which is optionally chlorine-substituted.

3. The method as claimed in claim 1 or 2, wherein
R¹ denotes methyl or chloromethyl.

4. The method as claimed in one or more of claims 1 to 3, wherein lipase from Pseudomonas is used.

## Revendications

1. Procédé de dédoublement enzymatique de racémates d'alcools racémiques, caractérisé en ce que l'on fait réagir un ester vinylique de formule I : 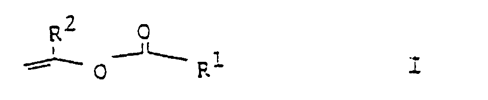 dans laquelle
R¹ représente l'atome d'hydrogène ou un groupe alkyle en C₁-C₁₈, lequel est éventuellement substitué par un halogène, un groupe phényle ou alcoxy (C₁-C₃)-alkyle (C₁-C₄), et
R² est un hydrogène ou un groupe méthyle,
en présence de lipases provenant du foie de porc, du pancréas de porc ainsi que de micro-organismes, tels que Candida, Mucor, Rhizopus, Penicillium, Aspergillus et Pseudomonas, avec un alcool répondent aux formules 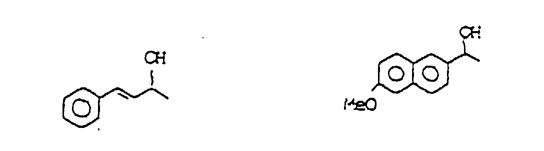 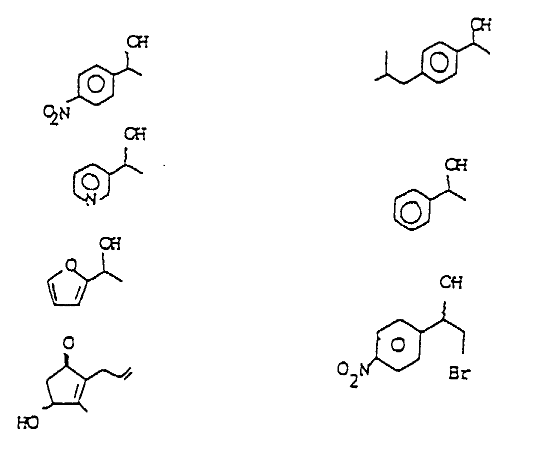 puis on isole ensuite l'alcool optiquement pur formé et on récupère éventuellement l'autre énantiomère à partir de l'ester restant.

2. Procédé selon la revendication 1, caractérisé en ce que R¹ représente un groupe alkyle en C₁-C₄ qui est éventuellement substitué par du chlore.

3. Procédé selon la revendication 1 ou 2, caractérisé en ce que :
R¹ représente un groupe méthyle ou chlorométhyle.

4. Procédé selon une ou plusieurs des revendications 1 à 3, caractérisé en ce qu'on utilise la lipase de Pseudomonas.
